# EUROPÄISCHE PATENTSCHRIFT

(11) **EP 3 536 285 B1**
(45) Veröffentlichungstag und Bekanntmachung des Hinweises auf die Patenterteilung: **05.05.2021**
(21) Anmeldenummer: 18020091.7
(22) Anmeldetag: 08.03.2018
(51) Int. Cl.: A61F 5/02, A41D 13/05, A41D 13/12, A61F 5/03

(54) **MODULARE ORTHESE, INSBESONDERE FÜR PATIENTEN MIT OSTEOPOROSE**
MODULAR ORTHOSIS, IN PARTICULAR FOR PATIENTS SUFFERING FROM OSTEOPOROSIS
ORTHÈSE MODULAIRE, EN PARTICULIER POUR PATIENTS ATTEINTS D'OSTÉOPOROSE

(43) Veröffentlichungstag der Anmeldung: 11.09.2019
(73) Patentinhaber: medi GmbH & Co. KG, 95448 Bayreuth (DE)
(72) Erfinder: Waldsich, Bettina, 95448 Bayreuth (DE); Pötzschner, Felix, 95448 Bayreuth (DE)

(56) Entgegenhaltungen:
- EP-A1- 1 902 691
- DE-A1-102016 201 270
- DE-T2- 69 019 740
- DE-U1- 29 720 475

## Beschreibung

Die vorliegende Erfindung bezieht sich auf eine modulare Orthese, insbesondere für Patienten mit Osteoporose, nach dem Oberbegriff des Anspruchs 1.

Derartiger Orthesen werden insbesondere zur Stabilisierung, Entlastung, Ruhigstellung, Aufrichtung, Führung und/ oder Korrektur von Gliedmaßen oder des Rumpfes von Patienten eingesetzt. Insbesondere Letztgenanntes spielt bei der Behandlung von Osteoporose, bei der durch die Abnahme der Knochendichte und in Folge durch Wirbelbrüche es zu einer Deformierung der Wirbelsäule und somit zu Fehlstellungen der Zwischenwirbelgelenke, Fehlfunktionen der Sehnen, Bänder und Muskulatur kommt, was Behinderungen im Alltag zur Folge hat, eine wichtige Rolle.

Eine derartige Rückenorthese zum Aufrichten der Wirbelsäule ist beispielsweise aus der EP 1 284 695 B1 bekannt.

Die bekannte Orthese ist in Form eines Kleidungsstücks, insbesondere als orthopädisch indizierte Unterwäsche ausgebildet, welche aus einem Baumwoll- oder Kunstfasermaterial besteht und verschiedene Stretchzonen aufweist, die im angelegten Zustand zur Stabilisierung der Wirbelsäule eine Spannung aufweisen. Die Spannung wird durch die unterschiedlichen Stretchzonen erzeugt. Die bekannte Orthese erlaubt ferner durch Anmodellieren einer speziellen Rückenschiene an die gekrümmte Wirbelsäule, die sich also von hinten an den Rücken anlegt, eine Aufrichtung der Wirbelsäule und somit des Oberkörpers des Patienten. Hierzu ist das orthopädische Kleidungsstück mit einer oben oder unten geöffneten länglichen Tasche zur Aufnahme der eng, elastisch oder mit geringem Spiel in dieser gehaltenen, die Wirbelsäule abstützenden, steifen Schiene ausgebildet.

Auch die EP 1 902 691 A1 offenbart eine derartige Orthese in Form eines Kleidungsstücks, insbesondere in Form eines Mieders. Um einen Rücken gerade aufzurichten, weist das Kleidungsstück eine Pelotte in Form einer Schiene, einen Querzug im Lendenbereich sowie einen Kreuzzug im Schulterbereich auf. Der Kreuzzug besteht aus zwei sich kreuzende Züge im Rückenbereich, welche in einem Brustbereich in Schulterstege des Mieders übergehen. Die Züge sind ebenfalls über Nahtverbindungen in das Kleidungsstück eingebracht. Die Einstellung des Grad der Entlastung der Wirbelsäule ist auch bei dieser bekannten Orthese nicht möglich.

Aus der DE 690 19 740 T2 ist eine Orthese in Form eines Korsetts bekannt, welches dazu dient einen Rücken zu stützen bzw. zu stabilisieren. Neben einem

Rückenstützabschnitt weist das Korsett zwei verstellbare Schulterträger auf, so dass das Korsett individuell für jeden Benutzer anpassbar ist.

Des Weiteren sind Orthesen zur Behandlung von Osteoporose bekannt, die neben einer die Wirbelsäule abstützenden Schiene, an Stelle eines normalen Kleidungsstückes, Befestigungsgurte, die nach Art von Rucksackträgern geführt und an der orthopädischen Schiene befestigt sind, aufweisen, um den Rücken eines Patienten aufzurichten.

Eine derartige Rückenorthese ist beispielsweise aus der EP 0 917 864 B1 bekannt.

Auch die DE 10 2016 201 270 A1 offenbart eine derartige Orthese mit einer die Wirbelsäule stützenden Schiene sowie mit einem daran befestigten Gurtsystem, bestehend aus einem Beckengurt sowie zwei einzelnen Schultergurten.

Abhängig von der therapeutisch notwendigen Behandlung, insbesondere dem Grad der notwendigen Stabilisierung, Entlastung, Ruhigstellung, Führung und/ oder Korrektur von Gliedmaßen, insbesondere des Rückens eines Patienten, durch die Orthese, erfolgt die Auswahl einer Orthese aus den aus dem Stand der Technik bekannten Orthesenvarianten.

Hierbei wird, insbesondere zu Beginn einer therapeutischen Behandlung, zu welchem Zeitpunkt ein hoher Grad an Stabilisierung, Entlastung, Ruhigstellung, Führung und/ oder Korrektur des Rückens erforderlich ist, bevorzugt die Rucksackvariante mit den mehreren Befestigungsgurten gewählt. Hierdurch ist es, insbesondere durch die mehreren Zug- und Druckelemente in Form von Gurten und Pelotten im Zusammenspiel mit der orthopädischen Schiene möglich, den Rücken eines Patienten aufzurichten. Die Rucksackvariante ermöglicht somit einen hohen Grad an Stabilisierung, Entlastung, Ruhigstellung, Führung und/ oder Korrektur von Gliedmaßen. Dadurch wird unter anderem das Lungenvolumen gesteigert und die häufig in der Osteoporose auftretende Kurzatmigkeit reduziert.

Als nachteilig erweist sich jedoch bei dieser Variante von Rückenorthesen, dass diese unter der Kleidung auftragen und als solche deutlich sichtbar bzw. für Dritte wahrnehmbar sind.

Speziell bei jüngeren und aktiveren Patienten, die sich eine diskrete therapeutische Behandlung, insbesondere diskrete Aufrichtung des Oberkörpers wünschen, finden die Rückenorthesen in Form von orthopädischen Kleidungsstücken mit integrierten Gurtzügelungen in Form von unterschiedlichen Stretchzonen Anwendung. Diese gewährleisten eine für Dritte nahezu nicht wahrnehmbare therapeutische Behandlung. Jedoch sind diese Orthesen, insbesondere zu Beginn der therapeutischen Behandlung, auf Grund dem eher niedrigen Grad an Stabilisierungsleistung und/ oder Korrektur der Körperhaltung ungeeignet. Die eingearbeitete Gurtzügelung wirkt zwar haltungsunterstützend, eine Aufrichtung des Rückens bzw. Korrektur des Oberkörpers im Ausmaß der Rucksackvarianten ist jedoch nicht möglich.Diese Orthesen eignen sich somit eher für einen späteren Zeitraum in der therapeutischen Behandlung, in der eine erste intensive Behandlung durch Orthesen in Form von Rucksäcken bereits erfolgreich war.

Zusammenfassend lässt sich festhalten, dass beide Varianten von Rückenorthesen, abhängig vom therapeutischen Behandlungszeitpunkt, ihre Vor- und Nachteile haben. Betrachtet man jedoch den gesamten therapeutischen Behandlungszeitraum ergeben sich aus dem Stand der Technik folgende Nachteile:

Als nachteilig für den Patienten erweist sich, dass eine diskrete therapeutische Behandlung während des gesamten therapeutischen Behandlungszeitraums mittels der aus dem Stand der Technik bekannten Rückenorthesen nicht möglich ist. Zumindest zu

Beginn und insbesondere je nach Behandlungsfortschritt wird zur Aufrichtung des Rückens bzw. Korrektur des Oberkörpers eine auftragende Rucksackvariante benötigt. Eine diskrete therapeutische Behandlung ist mit einer derartigen Orthese nicht möglich.

Einen weiteren Nachteil bildet die Tatsache, dass für eine möglichst diskrete Behandlung während des Behandlungszeitraums, zumindest zu einem späteren Zeitpunkt nachdem eine erste Behandlung durch die Rucksackvariante erfolgt, eine zweite Orthese in Form des Kleidungsstücks benötigt wird. Es fallen somit für den Patienten oder für die Krankenkasse, sofern eine vergütbare Orthese vorliegt, zusätzliche Kosten für die zweite diskrete Orthese an.

Der vorliegenden Erfindung liegt die Aufgabe zugrunde eine modulare Orthese bereitzustellen, welche die Nachteile aus dem Stand der Technik vermeidet, insbesondere den Grad der Stabilisierung, Entlastung, Ruhigstellung, Führung und/ oder Korrektur von Gliedmaßen durch die Orthese abhängig von der therapeutisch notwendigen Behandlung zu ermöglichen.

Gemäß einem Ausführungsbeispiel der modularen Orthese zur Behandlung von Osteoporose, besteht diese aus einem orthopädischen Kleidungsstück, insbesondere einer orthopädisch indizierten Unterwäsche, welches ein Textil mit verschiedenen Stretchzonen zur Entlastung und Korrektur der Wirbelsäule aufweist, wobei die Stretchzonen in Form von integrierten Gurtzügelungen als zwei die Schultern umgreifende Stretchzonen und zwei den Leib umgreifende Stretchzonen ausgebildet sind, und einer mit dem Kleidungsstück verbindbaren und die Wirbelsäule stützenden Schiene, die sich im getragenen Zustand entlang der Wirbelsäule erstreckt, wobei an dem Kleidungsstück und/ oder der Schiene ein den im gewünschten Grad der Entlastung und Korrektur der Wirbelsäule einstellbares Gurtsystem befestigt ist. Die Schiene selbst kann hierbei eine geradlinige Ausgestaltung, aber ebenso eine X, V oder Y-förmige Ausbildung, aufweisen.

Bevorzugt weist das Gurtsystem zumindest zwei die Schultern umgreifende Gurte und zumindest zwei den Leib umgreifende Gurte auf. Erstere umschließen hierbei bevorzugt den Übergangsbereich Brust- / Lendenwirbelsäule. Letztere, insbesondere der durch die zwei Gurte gebildete Rumpfgurt, umschließen bevorzugt das Becken. Die Gurte selbst sind hierbei bevorzugt gepolstert.

Gemäß einem zweiten Ausführungsbeispiel der modularen Orthese weisen die Gurte bevorzugt jeweils elastische und/ oder unelastische Gurtabschnitte auf. Zur Begrenzung der möglichen Dehnung in Längsrichtung der elastischen Gurtabschnitte des Gurtsystems, sind bevorzugt ein oder mehrere Dehnungsbegrenzungselemente vorgesehen. Diese können beispielsweise durch parallel zu dem elastischen

Gurtabschnitt an dem Gurtabschnitt angebrachte unelastische Gurtabschnitte ausgebildet sein.

Gemäß einem dritten Ausführungsbeispiel der modularen Orthese umfasst das Gurtsystem einen oder mehrere Längenversteller und/ oder kürzbare Gurtabschnitte, welche bevorzugt lösbar und/ oder wiederpositionierbar an der Orthese anbringbar sind. Hierdurch sind die Gurte bevorzugt in ihrer Länge individuell verstellbar und/ oder kürzbar, so dass diese abhängig von der Körperform des Patienten, welche tagsüber auf Grund der Zunahme von Essen und Trinken variiert, anpassbar sind.

Gemäß einem vierten Ausführungsbeispiel der modularen Orthese umfasst das Gurtsystem ferner eine Pelotte, nämlich einen sogenannten Druckkörper, bestehend aus zumindest zwei miteinander verbindbaren Halbteilen. An dieser Pelotte, welche bevorzugt als Bauchverschluss der Orthese ausgebildet ist, sind bevorzugt die die Schultern umgreifenden und/ oder den Leib umgreifenden Gurte lösbar oder unlösbar befestigt. Die Pelotte weist hierzu bevorzugt vier Fixierungspunkte für die mehreren Gurte des Gurtsystems auf. Die beiden Halbteile sowie die mehreren Gurte und die Pelotte sind bevorzugt durch eine Klettverschlussverbindung miteinander verbindbar.

Bevorzugt weist die Pelotte des Gurtsystems des Weiteren ein oder mehrere Zwischenstücke auf, an dem bzw. denen die miteinander verbindbaren Halbteile lösbar befestigbar sind. Durch die Ausbildung der Pelotte als verstellbaren Verschluss, wird eine Umfangseinstellung, insbesondere der den Leib umgreifenden Gurte, ermöglicht.

Die Befestigung des Gurtsystems an der modularen Orthese, insbesondere an dem Kleidungsstück und/ oder der Schiene erfolgt bevorzugt lösbar oder unlösbar durch eine stoffschlüssige Verbindung, insbesondere durch Löten, Schweißen, insbesondere HF Schweißen, Kleben und/ oder Vulkanisieren, durch eine formschlüssige Verbindung, insbesondere mittels einer Naht-, Druckknopf-, Magnet-, Klettverschluss-, Reißverschluss- und/ oder Haken und Ösenverbindung und/ oder durch ein oder mehrere Gurtführungselemente, insbesondere Laschen.

Auch bei der unlösbaren Befestigung der Gurte an der Orthese, insbesondere an dem Kleidungsstück und/ oder der Schiene, ist die Orthese modular aufgebaut, da durch eine Abtrennung der Gurte, insbesondere bei einer Nahtverbindung, oder einem Abschneiden der Gurte, insbesondere bei einem der weiteren unlösbaren Verbindungen, die Orthese abrüstbar ausgebildet ist. D.h. die Orthese kann von einer, insbesondere vollwertigen, Rückenorthese mit hohe Stützleistung, zu einer diskreten Orthese, im Wesentlichen bestehend aus nur einem in der Form eines Kleidungsstücks ausgebildeten Orthese mit oder ohne orthopädischen Schiene, umgebaut werden. Bei dieser Variante der Befestigung der Gurte ist eine Wiederaufrüstbarkeit der Orthese zu einer vollwertigen Rückenorthese, wie sie ursprünglich hergestellt bzw. ausgeliefert wurde, nicht möglich, was im Hinblick auf eine erfolgreiche therapeutische Behandlung auch nicht notwendig ist.

Gemäß einem weiteren Ausführungsbeispiel der modularen Orthese, weist das orthopädische Kleidungsstück zur lösbaren oder unlösbaren Aufnahme der orthopädischen Schiene eine Tasche, welche sich im getragenen Zustand entlang der Wirbelsäule erstreckt, auf. Die Tasche umfasst hierbei bevorzugt eine erste Öffnung zum wahlweise positionieren der Schiene in der Tasche und/ oder zumindest eine zweite Öffnung zum Hindurchführen eines oder mehrerer Gurte des Gurtsystems. Das Gurtsystem ist hierbei bevorzugt an der Tasche des Kleidungsstücks lösbar oder unlösbar befestigt.

Gemäß einem weiteren Ausführungsbeispiel der modularen Orthese, sind die verschiedenen Stretchzonen des orthopädischen Kleidungsstücks, welche eine eigene Gurtzügelung bilden, zumindest teilweise, insbesondere abschnittsweise, lösbar oder unlösbar von dem Textil des Kleidungsstücks ausgebildet. Hierdurch wird eine Einstellbarkeit der in das Kleidungsstück integrierten Gurtzügelung ermöglicht, um unterschiedliche Zug- und Druckwirkung auf den Körper des Patienten auszuüben.

Des Weiteren ist bevorzugt das orthopädische Kleidungsstück und/ oder die orthopädische Schiene, zur Vermeidung eines Verrutschens der Orthese am Körper des Patienten, an der Innenseite, also der dem Träger zugewandten Seite, zumindest teilweise mit einer rutschhemmenden Oberfläche versehen. Diese kann als Beschichtung der Textilinnenseite des Kleidungsstücks oder der Schiene, oder beispielsweise als eingestrickter rutschhemmender Bereich mittels eines rutschhemmenden Fadens, insbesondere Silikonfadens, ausgebildet sein. Ferner kann das Material der Schiene selbst rutschhemmende Eigenschaften aufweisen.

Das orthopädische Kleidungsstück selbst ist bevorzugt gemäß einem der vorherigen Ausführungsbeispiele in Form einer ein- oder mehrteiligen Unterwäsche, insbesondere als sogenannter Body, Radfahrerdress, Shirt, insbesondere T-Shirt, oder als Weste geschnitten.

Gemäß einem weiteren Ausführungsbeispiel der modularen Orthese ist das orthopädische Kleidungsstück zur lösbaren oder unlösbaren Aufnahme und Führung des Gurtsystems zumindest abschnittsweise mehrlagig, bevorzugt zweilagig, ausgebildet ist, so dass das Gurtsystem zumindest teilweise zwischen den beiden Lagen des Kleidungsstücks positionierbar ist. Die mehreren Gurte werden somit für den Patienten und Dritte weitestgehend unsichtbar in dem Kleidungsstück geführt, wodurch eine diskrete therapeutische Behandlung während des gesamten Behandlungszeitraums, insbesondere von Beginn an, gewährleistet werden kann.

Das vorliegende medizinische Hilfsmittel zeichnet sich durch eine Reihe erheblicher Vorteile aus.

Durch die erfindungsgemäße Orthese wird eine leistungsstarke Orthese in Form eines orthopädischen Kleidungsstücks bereitgestellt. Das einstellbare Gurtsystem mit Zug- und Druckelementen der Orthese ermöglicht einen hohen Grad an Stabilisierung, Entlastung, Ruhigstellung, Führung und/ oder Korrektur des Rumpfes. Die Orthese in Form eines Kleidungsstücks kann somit zur therapeutischen Behandlung von Beginn an verwendet werden.

Des Weiteren wird durch die Integration des Gurtsystems in das orthopädische Kleidungsstück, insbesondere zwischen den mehreren Schichten des Kleidungsstücks, zudem eine äußerst unauffällige Orthese zur Verfügung gestellt, welche eine diskrete therapeutische Behandlung über den gesamten Behandlungszeitraum ermöglicht.

Einen weiteren Vorteil der Erfindung bildet die Möglichkeit die modulare Orthese abhängig vom Behandlungsstadium bzw. -fortschritt abzurüsten und/ oder ggf. wieder aufzurüsten. Die Orthese kann alleine als orthopädisches Kleidungsstück mit den mehreren integrierten Gurtzügelungen, mit der orthopädischen Schiene gemeinsam und/ oder mit dem Gurtsystem getragen werden. Hierdurch ist es möglich, den Grad an Stabilisierungsleistung und/ oder Korrektur der Körperhaltung der Orthese individuell einzustellen bzw. auszuwählen. Für eine diskrete therapeutische Behandlung, insbesondere von Beginn an, wird nur eine einzige Orthese benötigt.

Nachfolgend wird die Erfindung anhand mehrerer Ausführungsbeispiele und in Verbindung mit den beigefügten Zeichnungen erläutert.

Dabei zeigt:
Figur 1 ein erstes Ausführungsbeispiel der modularen Orthese, insbesondere zur Behandlung von Osteoporose, mit einem orthopädischen Kleidungsstück in Form einer einteiligen Unterwäsche, insbesondere als sogenannter Body, zur Behandlung von Osteoporose mit einem daran befestigten Gurtsystem in einer Vorderansicht,
Figur 2 die Rückseite des ersten Ausführungsbeispiels der modularen Orthese, insbesondere das orthopädische Kleidungsstück in Form einer einteiligen Unterwäsche,
Figur 3 ein zweites Ausführungsbeispiel der modularen Orthese, insbesondere zur Behandlung von Osteoporose, mit einem orthopädischen Kleidungsstück in Form einer einteiligen Unterwäsche zur Behandlung von Osteoporose mit einem daran für Dritte unsichtbar angebrachten Gurtsystem in einer Vorderansicht,
Figur 4 die Rückseite des zweiten Ausführungsbeispiels der modularen Orthese, insbesondere des orthopädischen Kleidungsstücks in Form einer einteiligen Unterwäsche,
Figur 5 eine mit dem Kleidungsstück verbindbaren und die Wirbelsäule stützenden Schiene, die sich im getragenen Zustand entlang der Wirbelsäule erstreckt;

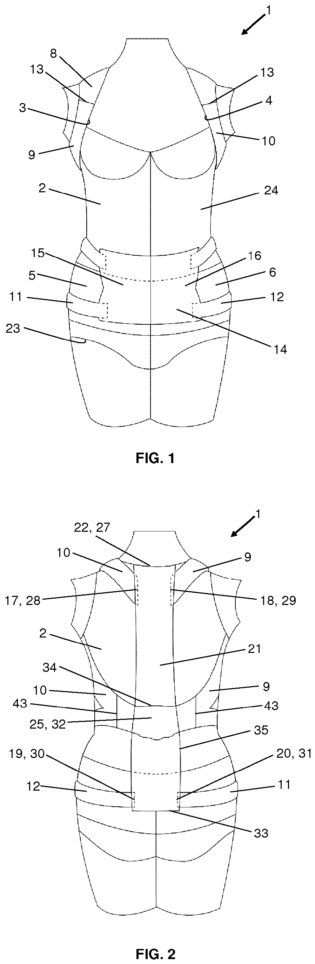

Figur 1 zeigt eine schematische Darstellung der modularen Orthese 1 mit einem orthopädischen Kleidungsstück 2 in Form einer einteiligen Unterwäsche 24 mit einem daran befestigten einstellbaren Gurtsystem 8 in einer Vorderansicht. Als einstellbares Gurtsystem 8 ist insbesondere ein an den Patienten anpassbares, insbesondere kürzbares, ein hinsichtlich der Dehnbarkeit einstellbares oder wahlweise positionierbares Gurtsystem zu verstehen. Die einteilige Unterwäsche 24 umfasst ein Textil mit verschiedenen Stretchzonen 3, 4, 5, 6 zur Entlastung und Korrektur der Wirbelsäule. Die Stretchzonen 3, 4, 5, 6 in Form von integrierten Gurtzügelungen sind hierbei bevorzugt als zwei die Schultern umgreifende Stretchzonen 3, 4 ausgebildet, welche in diesem Ausführungsbeispiel unterhalb der Schultergurte 9, 10 des Gurtsystems 8 angeordnet sind und dem Verlauf der Schultergurte 9, 10 folgen. Neben diesen Stretchzonen 3, 4 im Schulterbereich, umfasst das orthopädische Kleidungsstück 2 bevorzugt weitere Stretchzonen 5, 6 im Leibbereich. Diese sind ebenfalls weitestgehend in dieser Zeichnung nicht sichtbar unterhalb der den Leib umgreifenden Gurte 11, 12 des Gurtsystems 8 angeordnet. Beide Stretchzonen 3, 4, 5, 6 dienen zur Korrektur der Körperhaltung durch das Kleidungsstück 2. Diese Zonen werden bevorzugt aus unterschiedlich elastischen Materialien gefertigt. Dabei werden bevorzugt gegenüber dem restlichen Kleidungsstück 24 weniger elastische Materialen verwendet.

Das an dem Kleidungsstück 2 und/ oder der Schiene 7 befestigte Gurtsystem 8 zur Einstellung des Grad der Entlastung und Korrektur der Wirbelsäule besteht aus den zwei die Schultern umgreifende Gurte 9, 10, die im Übergangsbereich der Brustwirbelsäule und Lendenwirbelsäule den Rumpf umschließen, sowie zwei den Leib umgreifende Gurte 11, 12. Die Gurte 9, 10 verlaufen, kommend von der Rückseite der Orthese 1, nach Art von Rucksackträgern über die Schultern und unter den Achseln hindurch zurück an die Rückseite, werden dort bevorzugt mittels der Schiene 7, oder zumindest einem Umlenkgurt 25 mit beabstandeten Umlenkelementen, welcher an der Schiene 7 oder dem Kleidungsstück 2 befestigt ist, umgelenkt und zu der Pelotte 14 geführt, an der diese bevorzugt lösbar, insbesondere mittels Klettverschlusselementen, befestigt sind. Das Gurtsystem 8 umfasst somit eine geschlossene Gurtführung, d.h. die mehreren Gurte 9, 10, 11, 12 und die zumindest eine Pelotte 14 sind direkt miteinander verbunden. Insbesondere sind die mehreren Endabschnitte der einzelnen Bauteile überlappend aneinander angeordnet. D.h. diese sind wahlweise übereinander oder untereinander aneinander angebracht. Die bevorzugt gepolsterten Gurte 9, 10, 11, 12 selbst sind hierbei bevorzugt individuell kürzbar, je nach Patientengröße, oder weisen einen oder mehrere Längenversteller 13 auf. Bevorzugt wird das orthopädische Kleidungsstück 2 sowie das Gurtsystem 8 in verschieden Leibumfängen und Rückenhöhen bereit gestellt, wobei die kürzbaren Gurtsysteme zur Feinjustierung der Orthese 2 dienen. Die Gurte 9, 10, 11, 12 selbst sind bevorzugt aus einem im Wesentlichen unelastischen Material gefertigt. Alternativ ist es möglich, die im Wesentlichen unelastischen Gurte 9, 10, 11, 12 mit einem oder mehreren elastischen Gurtabschnitten auszubilden. Hierbei werden bevorzugt an den bzw. parallel zu den elastischen Gurtabschnitten Dehnungsbegrenzungselemente angeordnet, um die Dehnung der Gurte 9, 10, 11, 12 zu begrenzen.

Die Pelotte 14 des Gurtsystems 8 der Orthese 1 besteht bevorzugt aus zumindest zwei miteinander verbindbaren Halbteilen 15, 16, welche einen Verschluss für das Gurtsystem 8 bilden. Bevorzugt weist die Pelotte 14 ein oder mehrere nicht gezeigte Zwischenstücke auf, an dem bzw. denen die miteinander verbindbaren Halbteile 15, 16 lösbar befestigbar sind. Hierdurch wird eine Umfangseinstellung des Gurtsystems 8 im Rumpfbereich ermöglicht. Die Innenseite des Textils der Orthese 1 ist bevorzugt mit einer rutschhemmenden Oberfläche 23 verstehen, so dass ein verrutschen der Orthese 1 am Körper eines Patienten vermieden wird.

Figur 2 zeigt die Rückseite der modularen Orthese 1 aus Figur 1. An dem Kleidungsstück 2 der Orthese 1 ist eine Tasche 21 zur lösbaren oder unlösbaren Aufnahme der orthopädischen Schiene 7 angebracht. Diese Tasche 21 ist bevorzugt auf das Kleidungsstück 2 aufgenäht. Die Tasche 21 erstreckt sich im getragenen Zustand entlang der Wirbelsäule. Zur Aufnahme der orthopädischen Schiene 7, welche sich bevorzugt ca. vom Kreuzbein bis zur Oberkante der Schulterblätter erstreckt, weist die Tasche 21 zumindest eine erste Öffnung 22 auf. Diese ist bevorzugt am oberen Ende 27 oder besonders bevorzugt am unteren Ende der Tasche 21 angebracht. In diesem ersten Ausführungsbeispiel ist das im Grad der Entlastung und Korrektur der Wirbelsäule einstellbare Gurtsystem 8 ausschließlich an der Tasche 21 des orthopädischen Kleidungsstück 2 befestigt. Die Tasche 21 weist hierzu fünf Fixierungspunkte 28, 29, 30, 31, 32 für die mehreren Gurte 9, 10, 11, 12 auf. Alternativ können die Gurte 9, 10, 11, 12 auch direkt an der Schiene 7, insbesondere an deren oberen und unteren Ende sowie im mittleren Bereich der Schiene 7, befestigt sein. Hierzu würde die Tasche 21 weitere Öffnungen zum Hindurchführen der Gurte des Gurtsystems aufweisen. Im vorliegenden Ausführungsbeispiel sind die Schultergurte 9, 10 an dem oberen Ende 27 und die den Leib umgreifenden Gurte 11, 12 an dem unteren Ende 33 der Tasche 21 des Kleidungsstück 2 für den Patienten unlösbar mittels einer stoffschlüssigen Verbindung, vorzugsweise mittels einer Nahtverbindung 17, 18, 19, 20 angebracht. Weitere unlösbare Verbindungen, wie zum Beispiel Löten, Schweißen, Kleben und/ oder Vulkanisieren wären ebenfalls möglich.

Bevorzugt wird zur Verbindung des Gurtsystems 8 mit der Orthese 1, insbesondere der vier Gurte 9, 10, 11, 12, eine auftrennbare Nahtverbindung 17, 18, 19, 20 verwendet, welche es ermöglicht, das Gurtsystem 8 von der Orthese 1, durch das Auftrennen der Nähte 17, 18, 19, 20 zu lösen und die Orthese 1 je nach Behandlungsfortschritt abzurüsten. Die Nähte 17, 18, 19, 20 liegen hierbei bevorzugt in der umlaufenden Naht 35 der Tasche 21, so dass nach einem Auftrennen der Nähte 17, 18, 19, 20 keine Spuren der ursprünglichen Verbindung mehr sichtbar sind.

Der mittlere Gurt, insbesondere Umlenkgurt 25 für die zwei Schultergurte 9, 10 des Gurtsystems 8 der Orthese 1, ist bevorzugt mittels einer lösbaren Verbindung 32 an der Tasche 21 der Orthese 1 befestigt. Hierdurch sind die Gurte 9, 10 höhenverstellbar an der Orthese 1 befestigbar, so dass eine Umlenkung der Schultergurte 9, 10 individuell an den Patienten angepasst werden kann. Die Umlenkung der Gurte 9, 10 erfolgt hierbei bevorzugt in Umfangsrichtung beabstandet von der Schiene 7, also neben der Schiene 7, insbesondere in den Positionen 43. Alternativ kann die Umlenkung auch direkt im Bereich der Schiene 7 oder durch die Schiene 7, insbesondere durch darin eingebrachte Umlenkschlitze, erfolgen. Die variable Umlenkung ermöglicht eine sogenannte Größeneinstellung der Orthese 1 abhängig vom Patienten. Hierzu weisen die mehreren Gurte 9, 10, 11, 12 des Weiteren bevorzugt einen oder mehreren Längenversteller 13 und/ oder kürzbare Gurtabschnitte auf. Bei Letztgenannten sind bevorzugt lösbare Befestigungsmittel am Ende der Gurte 9, 10, 11, 12 vorgesehen, um nach kürzen des Gurtsystems eine Befestigung der gekürzten Gurte zu ermöglichen. Hierzu werden bevorzugt sogenannte Y-Klettverschlusselemente verwendet, deren erstes Ende den gekürzten Gurt 9, 10, 11, 12 maulartig umgreifen und deren zweites flache Ende auf einer weiteren Fläche aufklettbar ist.

Alternativ wäre es auch denkbar, um eine Wiederaufrüstbarkeit der Orthese 1 zu gewährleisten, die Nahtverbindungen 17, 18, 19, 20 durch für den Anwender lösbare Verbindungen zu ersetzen. Hierzu wären besonders Klettverschlussverbindungen geeignet. Ebenso wären Druckknopf-, Magnet-, Reißverschluss- und/ oder Haken und Ösenverbindung möglich.

Das in Figur 1 und 2 gezeigte Ausführungsbeispiel einer Rückenorthese 1 zeigt, dass der modulare Aufbau der Orthese 1, eine individuelle Anpassung der Leistungsfähigkeit der Orthese, nämlich an den therapeutisch notwendigen Rahmen, ermöglicht, um eine möglichst diskrete Behandlung eines Patienten zu gewährleisten. Des Weiteren ermöglicht diese Variante eine möglichst unzugängliche Ausführung des Gurtsystems. Ein versehentliches verstellen der Gurte wird vermieden. Auch kann das Gurtsystem dadurch derart ausgebildet werden, dass dieses für den Anwender nicht verstellbar ist.

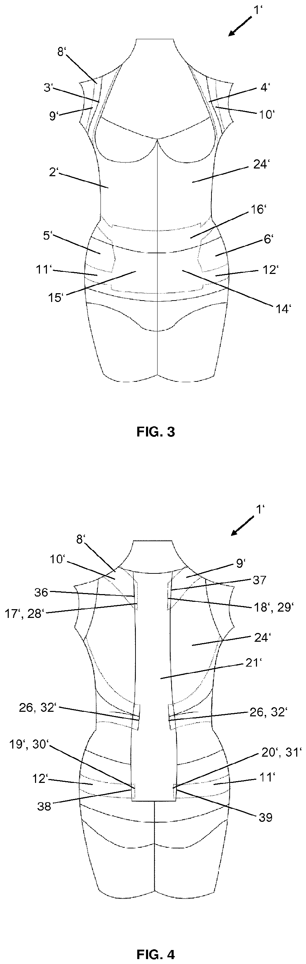

Figur 3 zeigt ein zweites Ausführungsbeispiel der modularen Orthese 1', insbesondere des orthopädischen Kleidungsstücks 2', mit einem daran für Dritte im Wesentlichen unsichtbar angebrachten Gurtsystem 8'. Das orthopädische Kleidungsstück 2' ist in diesem Ausführungsbeispiel ebenfalls als bevorzugt einteilig orthopädisch indizierte Unterwäsche 24' ausgebildet, welche ein Textil mit verschiedenen Stretchzonen 3', 4', 5', 6' zur Entlastung und Korrektur der Wirbelsäule aufweist. Diese integrierte Gurtzügelung ist, wie zu Figur 1 beschrieben, durch zwei die Schultern umgreifende Stretchzonen 3', 4' sowie zwei den Leib umgreifende Stretchzonen 5', 6' ausgebildet, welche im Zusammenspiel die Körperhaltung des Patienten korrigieren. Die Rumpfzügelung 5',6' erstreckt sich bevorzugt umlaufend um das gesamte Kleidungsstück 2' und baut durch deren kompressiven Eigenschaft einen zirkulären Kompressionsdruck auf den Körper des Patienten auf, so dass die Orthese 2' eine den Körper aufrichtende Eigenschaft aufweist. Die Schultergurte 3', 4' bewirken eine weitere Aufrichtung des Oberkörpers. Alternativ zu der integrierten Variante dieser Gurtzügelung 3', 4', 5', 6', kann diese auch zumindest teilweise bzw. abschnittsweise lösbar von dem Textil des Kleidungsstücks 2' ausgebildet sein. Die lösbaren Abschnitte sind hierbei wahlweise an dem Körper unter mehr oder weniger Zug an dem Kleidungsstück 2' positionierbar, so dass eine individuelle Einstellung des durch die Gurte erzeugten Kompressionsdruckes oder Zugwirkung möglich ist.

Neben der integrierten Gurtzügelung 3', 4', 5`, 6' weist die Orthese 1' des Weiteren ein an dem Kleidungsstück 2' und der Schiene 7' ein den Grad der Entlastung und Korrektur der Wirbelsäule einstellbares Gurtsystem 8' auf. Im Gegensatz zu der in den Figuren 1 und 2 beschrieben Ausführungsbeispiel ist gemäß Figur 3 und Figur 4 das Gurtsystem 8' an der Innenseite des orthopädischen Kleidungsstücks 2' bzw. zumindest teilweise zwischen zwei oder mehreren Lagen des Kleidungsstücks 2' zumindest abschnittsweise von außen betrachtet unsichtbar positioniert. Auch in diesem Ausführungsbeispiel umfasst das Gurtsystem 8' zwei die Schultern umgreifende Gurte 9', 10', zumindest zwei den Leib umgreifende Gurte 11', 12' sowie zumindest eine Verschlusspelotte 14', bestehend aus zumindest zwei miteinander verbindbaren Halbteilen 15', 16', auf. Im Bereich der integrierten Stretchenzonen 3', 4', 5', 6' erfolgt die Gurtführung 9', 10', 11', 12' bevorzugt außerhalb dieser Zonen 3', 4', 5', 6', also nicht zwischen Kleidungsstück 2' und Patienten, um unangenehme Druckstellen, welche durch die kompressiven Zonen 3', 4', 5', 6' erzeugt werden, zu vermeiden. Hierbei bildet beispielsweise die innere Lage des zwei- oder mehrlagigen Kleidungsstück 2' die Stretchzone, wobei eine äußere Lage dazu dient das Gurtsystem 8' abzudecken.

In Figur 4 ist die Rückseite des Ausführungsbeispiels der modularen Orthese 1' aus Figur 3 gezeigt. Die Gurte 9', 10', 11', 12' des Gurtsystems 8' der Orthese 1' sind bevorzugt an der entlang der Wirbelsäule verlaufenden orthopädischen Schiene 7 befestigt und erstrecken sich hierzu durch das orthopädische Kleidungsstück 2', also durch Öffnungen 36, 37, 38, 39 in der Tasche 21'. Die Befestigung der Gurte 9', 10', 11', 12' an der Schiene 7 an deren oberen und unteren Ende, insbesondere an den Fixierungspunkten 28', 29', 30', 31', erfolgt bevorzugt durch eine formschlüssige Verbindung 17', 18', 19' 20', insbesondere mittels einer Naht-, Druckknopf-, Magnet-, Klettverschluss-, Reißverschluss- und/ oder Haken und Ösenverbindung. Die Umlenkung der Schultergurte 9', 10' an dem fünften Fixierungspunkt 32' erfolgt bevorzugt durch Schlitze 26 in der Schiene 7. Alternativ zu der geradlinig an der Wirbelsäule verlaufenden Schiene 7, kann ebenso eine X, V oder Y-förmig an der Wirbelsäule entlang in vertikaler Richtung verlaufende Schiene vorgesehen sein. Des Weiteren ist betreffend der Schultergurte 9', 10' eine im mittleren Bereich der Schiene 7 über Kreuz durchlaufende Gurtführung denkbar. Ein Umlenkgurt wie in Figur 2 beschrieben wird hierbei nicht benötigt.

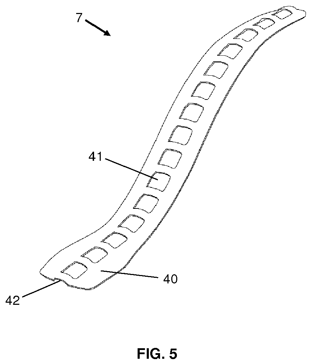

In Figur 5 ist eine mit dem Kleidungsstück 2, 2' verbindbare und die Wirbelsäule stützende Schiene 7, die sich im getragenen Zustand entlang der Wirbelsäule erstreckt, gezeigt. Die Schiene 7 weist eine dem Verlauf der Wirbelsäule folgend gekrümmte Kontur auf. An ihrem unteren Ende 40 ist sie bevorzugt verbreitert ausgeführt. Um die Dorfortsätze der Wirbelsäule zu überbrücken, weist sie des Weiteren eine oder mehrere Aussparungen 41 auf. Zur Gewichtsreduzierungen sind ebenso bevorzugt Ausnehmungen 42 vorhanden. Das Material der Schiene 7 ist bevorzugt derart gewählt, dass sich die Schiene 7 individuell an die Form der Wirbelsäule des Patienten anmodellieren lässt. Alternativ kann die Schiene 7 auch vorgeformt sein. Je nach Ausführung kann die Schiene 7 unlösbar von der Orthese 1, 1', also fest in der Tasche 21, 21' der Orthese 1,1' eingebracht, oder lösbar, also entnehmbar von der Tasche 21, 21' der Orthese 1,1' ausgebildet sein.

Die Erfindung ist nicht auf die beschriebenen Ausführungsbeispiele beschränkt, sondern umfasst alle Ausführungen, die das prinzipielle, sinngemäße Funktionsprinzip der Erfindung anwenden oder beinhalten, soweit sie von den Ansprüchen umfasst werden.

## Patentansprüche

1. Modulare Orthese (1, 1'), insbesondere für Patienten mit Osteoporose, bestehend aus einem orthopädischen Kleidungsstück (2, 2'), insbesondere einer orthopädisch indizierten Unterwäsche (24, 24'), welches ein Textil mit verschiedenen Stretchzonen (3, 4, 5, 6; 3', 4', 5', 6') zur Entlastung und Korrektur der Wirbelsäule aufweist, wobei die Stretchzonen (3, 4, 5, 6; 3', 4', 5', 6') in Form von integrierten Gurtzügelungen als zwei die Schultern umgreifende Stretchzonen (3, 4; 3', 4') und zwei den Leib umgreifende Stretchzonen (5, 6; 5', 6') ausgebildet sind, und einer mit dem Kleidungsstück (2, 2') verbindbaren und die Wirbelsäule stützenden Schiene (7), die sich im getragenen Zustand entlang der Wirbelsäule erstreckt, wobei an dem Kleidungsstück (2, 2') und/ oder der Schiene (7) ein den Grad der Entlastung und Korrektur der Wirbelsäule einstellbares Gurtsystem (8, 8') befestigt ist.

2. Modulare Orthese (1, 1') zur Behandlung von Osteoporose nach Anspruch 1, wobei das Gurtsystem (8, 8') zumindest zwei die Schultern umgreifende Gurte (9, 10; 9', 10') und zumindest zwei den Leib umgreifende Gurte (11, 12; 11', 12') aufweist.

3. Modulare Orthese (1, 1') zur Behandlung von Osteoporose nach Anspruch 2, wobei die mehreren Gurte (9, 10, 9', 10'; 11, 12, 11', 12') des Gurtsystems (8, 8') jeweils elastische und/ oder unelastische Gurtabschnitte aufweisen.

4. Modulare Orthese (1, 1') zur Behandlung von Osteoporose nach Anspruch 3, wobei zur Begrenzung der Dehnung der elastischen Gurtabschnitte das Gurtsystem (8, 8') ein oder mehrere Dehnungsbegrenzungselemente aufweist.

5. Modulare Orthese (1, 1') zur Behandlung von Osteoporose nach einem der vorherigen Ansprüche, wobei das Gurtsystem (8, 8') zur Längenverstellung der mehreren Gurte (9, 10, 9', 10'; 11, 12, 11', 12') einen oder mehrere Längenversteller (13) und/ oder kürzbare Gurtabschnitte aufweist.

6. Modulare Orthese (1, 1') zur Behandlung von Osteoporose nach einem der vorherigen Ansprüche, wobei das Gurtsystem (8, 8') zumindest eine Pelotte (14, 14'), bestehend aus zumindest zwei miteinander verbindbaren Halbteilen (15, 16; 15', 16') aufweist, an der die die Schultern umgreifenden und/ oder den Leib umgreifenden Gurte (9, 10, 9', 10'; 11, 12, 11', 12') lösbar oder unlösbar befestigbar sind.

7. Modulare Orthese (1, 1') zur Behandlung von Osteoporose nach Anspruch 6, wobei die Pelotte (14, 14') des Gurtsystems (8, 8') ein oder mehrere Zwischenstücke aufweist, an dem bzw. denen die miteinander verbindbaren Halbteile (15, 16; 15', 16') lösbar oder unlösbar befestigbar sind.

8. Modulare Orthese (1, 1') zur Behandlung von Osteoporose nach einem der vorherigen Ansprüche, wobei das Gurtsystem (8, 8') an dem Kleidungsstück (2, 2') und/ oder der Schiene (7) durch eine stoffschlüssige Verbindung, insbesondere durch Löten, Schweißen, Kleben und/ oder Vulkanisieren, durch eine formschlüssige Verbindung (17, 18, 19, 20; 17', 18', 19', 20'), insbesondere mittels einer Naht-, Druckknopf-, Magnet-, Klettverschluss-, Reißverschluss- und/ oder Haken und Ösenverbindung und/ oder durch ein oder mehrere Gurtführungselemente, insbesondere Laschen, lösbar oder unlösbar befestigt ist.

9. Modulare Orthese (1, 1') zur Behandlung von Osteoporose nach einem der vorherigen Ansprüche, wobei das orthopädische Kleidungsstück (2, 2') zur lösbaren oder unlösbaren Aufnahme der Schiene (7) eine Tasche (21, 21'), welche sich im getragenen Zustand entlang der Wirbelsäule erstreckt, aufweist.

10. Modulare Orthese (1, 1') zur Behandlung von Osteoporose nach Anspruch 9, wobei die Tasche (21, 21') des orthopädischen Kleidungsstücks (2, 2') zumindest eine erste Öffnung (22) zum wahlweise positionieren der Schiene (7) in der Tasche (21, 21') und/ oder zumindest eine zweite Öffnung (36, 37, 38, 39) zum Hindurchführen eines oder mehrerer Gurte (9', 10'; 11', 12') des Gurtsystems (8, 8') aufweist.

11. Modulare Orthese (1) zur Behandlung von Osteoporose nach Anspruch 9, wobei das Gurtsystem (8, 8') an der Tasche (21) des Kleidungsstücks (2) lösbar oder unlösbar befestigt ist.

12. Modulare Orthese (1, 1') zur Behandlung von Osteoporose nach einem der vorherigen Ansprüche, wobei die verschiedenen Stretchzonen (3, 4, 5, 6; 3', 4', 5', 6') des orthopädischen Kleidungsstücks (2, 2') zumindest teilweise, insbesondere abschnittsweise, lösbar oder unlösbar von dem Textil des Kleidungsstücks (2, 2') ausgebildet sind.

13. Modulare Orthese (1) zur Behandlung von Osteoporose nach einem der vorherigen Ansprüche, wobei das orthopädische Kleidungsstück (2) und/ oder die orthopädische Schiene (7) an der Innenseite, also der dem Patienten zugewandten Seite, zumindest teilweise mit einer rutschhemmenden Oberfläche (23) verstehen ist.

14. Modulare Orthese (1, 1') zur Behandlung von Osteoporose nach einem der vorherigen Ansprüche, wobei das orthopädische Kleidungsstück (2, 2') in Form einer ein- oder mehrteiligen Unterwäsche, insbesondere als sogenannter Body (24, 24'), Radfahrerdress, Shirt, insbesondere T-Shirt, oder als Weste geschnitten ist.

15. Modulare Orthese (1') zur Behandlung von Osteoporose nach einem der vorherigen Ansprüche, wobei das orthopädische Kleidungsstück (2') zur lösbaren oder unlösbaren Aufnahme und Führung des Gurtsystems (8') zumindest abschnittsweise mehrlagig, bevorzugt zweilagig, ausgebildet ist, so dass das Gurtsystem (8') zumindest teilweise zwischen den beiden Lagen des Kleidungsstücks (2') positionierbar ist.

## Claims

1. A modular orthosis (1, 1'), in particular for patients with osteoporosis, consisting of an orthopaedic garment (2, 2'), in particular an orthopaedically indicated undergarment (24, 24') which has a textile with various stretch zones (3, 4, 5, 6; 3', 4', 5', 6') for relieving and correcting the spine, wherein the stretch zones (3, 4, 5, 6; 3', 4', 5', 6') are designed in the form of integrated support strappings as two stretch zones (3, 4; 3', 4') encompassing the shoulders and two stretch zones (5, 6; 5', 6') encompassing the abdomen, and a splint (7), which is connectable to the garment (2, 2'), supports the spine, and, when the garment is worn, extends along the spine, wherein a belt system (8, 8') able to adjust the level of relief and correction of the spine is fastened to the garment (2, 2') and/or the splint (7) .

2. The modular orthosis (1, 1') for treating osteoporosis according to claim 1, wherein the belt system (8, 8') has at least two belts (9, 10; 9', 10') encompassing the shoulders and at least two belts (11, 12; 11', 12') encompassing the abdomen.

3. The modular orthosis (1, 1') for treating osteoporosis according to claim 2, wherein the plurality of belts (9, 10, 9', 10'; 11, 12, 11', 12') of the belt system (8, 8') each have elastic and/or inelastic belt portions.

4. The modular orthosis (1, 1') for treating osteoporosis according to claim 3, wherein, in order to delimit the elongation of the elastic belt portions, the belt system (8, 8') has one or more elongation-limiting elements.

5. The modular orthosis (1, 1') for treating osteoporosis according to one of the preceding claims, wherein, to adjust the length of the plurality of belts (9, 10, 9', 10'; 11, 12, 11', 12'), the belt system (8, 8') has one or more length adjusters (13) and/or has belt portions that can be shortened.

6. The modular orthosis (1, 1') for treating osteoporosis according to one of the preceding claims, wherein the belt system (8, 8') has at least one pad (14, 14') consisting of at least two interconnectable half parts (15, 16; 15', 16'), to which the belts (9, 10, 9', 10'; 11, 12, 11', 12') encompassing the shoulders and/or encompassing the abdomen are fastenable detachably or non-detachably.

7. The modular orthosis (1, 1') for treating osteoporosis according to claim 6, wherein the pad (14, 14') of the belt system (8, 8') has one or more intermediate pieces to which the half part(s) (15, 16; 15', 16') connectable to one another are fastenable detachably or non-detachably.

8. The modular orthosis (1, 1') for treating osteoporosis according to one of the preceding claims, wherein the belt system (8, 8') is fastened detachably or non-detachably to the garment (2, 2') and/or the splint (7) by an integrally bonded connection, in particular by soldering, welding, adhesive bonding and/or vulcanisation, by an interlocking connection (17, 18, 19, 20; 17', 18', 19', 20'), in particular by means of a seam, press studs, magnetic connection, hook-and-loop connection, zipper and/or hook-and-eye connection and/or by one or more belt guide elements, in particular straps.

9. The modular orthosis (1, 1') for treating osteoporosis according to one of the preceding claims, wherein the orthopaedic garment (2, 2'), to detachably or non-detachably receive the splint (7), has a pocket (21, 21') which extends along the spine when the garment is worn.

10. The modular orthosis (1, 1') for treating osteoporosis according to claim 9, wherein the pocket (21, 21') of the orthopaedic garment (2, 2') has at least one first opening (22) for selectively positioning the splint (7) in the pocket (21, 21') and/or at least one second opening (36, 37, 38, 39) for guiding through one or more belts (9', 10'; 11', 12') of the belt system (8, 8').

11. The modular orthosis (1) for treating osteoporosis according to claim 9, wherein the belt system (8, 8') is fastened detachably or non-detachably to the pocket (21) of the garment (2).

12. The modular orthosis (1, 1') for treating osteoporosis according to one of the preceding claims, wherein the various stretch zones (3, 4, 5, 6; 3', 4', 5', 6') of the orthopaedic garment (2, 2') are designed to be detachable or non-detachable from the textile of the garment (2, 2') at least in part, in particular in portions.

13. The modular orthosis (1) for treating osteoporosis according to one of the preceding claims, wherein the orthopaedic garment (2) and/or the orthopaedic splint (7) is provided on the inner side, that is to say the side facing the patient, at least in part with an anti-slip surface (23).

14. The modular orthosis (1, 1') for treating osteoporosis according to one of the preceding claims, wherein the orthopaedic garment (2, 2') is cut in the form of a one-part or multi-part undergarment, in particular as what is known as a bodysuit (24, 24'), cycling suit, shirt, in particular t-shirt, or as a vest.

15. The modular orthosis (1') for treating osteoporosis according to one of the preceding claims, wherein the orthopaedic garment (2'), in order for the belt system (8') to be detachably or non-detachably received and guided, is designed at least in portions in a number of layers, preferably two layers, such that the belt system (8') is positionable at least in part between the two layers of the garment (2').

## Revendications

1. Orthèse modulaire (1, 1'), destinée notamment à des patients atteints d'ostéoporose, constituée d'un vêtement orthopédique (2, 2'), notamment d'un sous-vêtement (24, 24') à index orthopédique, lequel comporte un textile doté de différentes zones de stretch (3, 4, 5, 6 ; 3', 4', 5', 6'), pour décharger et corriger la colonne vertébrale, les zones de stretch (3, 4, 5, 6 ; 3', 4', 5', 6') étant conçues sous la forme de renforts par sangle intégrés comme deux zones de stretch (3, 4 ; 3', 4') entourant les épaules et deux zones de stretch (5, 6 ; 5', 6') entourant le corps et d'un rail (7) susceptible d'être assemblé avec le vêtement (2, 2') et assurant le soutien de la colonne vertébrale, qui à l'état porté, s'étend le long de la colonne vertébrale, sur le vêtement (2, 2') et/ou sur le rail (7) étant placé un système de sangles (8, 8') réglant le taux de décharge et de correction.

2. Orthèse modulaire (1, 1'), destinée à traiter l'ostéoporose selon la revendication 1, le système de sangles (8, 8') comportant au moins deux sangles (9, 10 ; 9', 10') entourant les épaules et au moins deux sangles (11, 12 ; 11', 12') entourant le corps.

3. Orthèse modulaire (1, 1'), destinée à traiter l'ostéoporose selon la revendication 2, les plusieurs sangles (9, 10, 9', 10' ; 11, 12, 11', 12') du système de sangles (8, 8') comportant chacune des parties de sangle élastiques et/ou non élastiques.

4. Orthèse modulaire (1, 1'), destinée à traiter l'ostéoporose selon la revendication 3, pour limiter l'extension des parties de sangle élastiques, le système de sangles (8, 8') comportant un ou plusieurs éléments limiteurs d'extension.

5. Orthèse modulaire (1, 1'), destinée à traiter l'ostéoporose selon l'une quelconque des revendications précédentes, pour le réglage en longueur des plusieurs sangles (9, 10, 9', 10' ; 11, 12, 11', 12'), le système de sangles (8, 8') comportant un ou plusieurs régleurs de longueur (13) et/ou des parties de sangle raccourcissables.

6. Orthèse modulaire (1, 1'), destinée à traiter l'ostéoporose selon l'une quelconque des revendications précédentes, le système de sangles (8, 8') comportant au moins une pelote (14, 14'), constituée d'au moins deux demies parties (15, 16 ; 15', 16') susceptibles d'être assemblées l'une avec l'autre, sur laquelle les sangles (9, 10, 9', 10' ; 11, 12, 11', 12') entourant les épaules et/ou le corps sont susceptibles d'être fixées de manière amovible ou inamovible.

7. Orthèse modulaire (1, 1'), destinée à traiter l'ostéoporose selon la revendication 6, la pelote (14, 14') du système de sangles (8, 8') comportant une ou plusieurs pièces intermédiaires, sur laquelle ou lesquelles les demies parties (15, 16 ; 15', 16') susceptibles d'être assemblées sont susceptibles d'être fixées de manière amovible ou inamovible.

8. Orthèse modulaire (1, 1'), destinée à traiter l'ostéoporose selon l'une quelconque des revendications précédentes, le système de sangles (8, 8') étant fixé de manière amovible ou inamovible sur le vêtement (2, 2') et/ou sur le rail (7) par un assemblage par matière, notamment par brasage, par soudage, par collage et/ou par vulcanisation, par un assemblage par complémentarité de forme (17, 18, 19, 20 ; 17', 18', 19', 20'), notamment au moyen d'un assemblage par couture, par bouton-pression, par aimant, par bande auto-agrippante, par fermeture-éclair et/ou par crochet et œillet et/ou par un ou plusieurs éléments de guidage de sangle, notamment des pattes.

9. Orthèse modulaire (1, 1'), destinée à traiter l'ostéoporose selon l'une quelconque des revendications précédentes, pour recevoir de manière amovible ou inamovible le rail (7), le vêtement orthopédique (2, 2') comportant une poche (21, 21'), qui à l'état porté s'étend le long de la colonne vertébrale.

10. Orthèse modulaire (1, 1'), destinée à traiter l'ostéoporose selon la revendication 9, la poche (21, 21') du vêtement orthopédique (2, 2') comportant au moins une première ouverture (22), pour le positionnement au choix du rail (7) dans la poche (21, 21') et/ou au moins une deuxième ouverture (36, 37, 38, 39) pour y faire passer une ou plusieurs sangles (9', 10', 11', 12') du système de sangles (8, 8').

11. Orthèse modulaire (1), destinée à traiter l'ostéoporose selon la revendication 9, le système de sangles (8, 8') étant fixé de manière amovible ou inamovible sur la poche (21) du vêtement (2).

12. Orthèse modulaire (1, 1'), destinée à traiter l'ostéoporose selon l'une quelconque des revendications précédentes, les différentes zones de stretch (3, 4, 5, 6 ; 3', 4', 5', 6') du vêtement orthopédique (2, 2')étant conçues au moins en partie, notamment par endroits de manière amovible ou inamovible par le textile du vêtement (2, 2').

13. Orthèse modulaire (1), destinée à traiter l'ostéoporose selon l'une quelconque des revendications précédentes, sur la face intérieure, donc sur la face tournée vers le patient, le vêtement orthopédique (2) et/ou le rail orthopédique (7) étant muni au moins en partie d'une surface (23) antidérapante.

14. Orthèse modulaire (1, 1'), destinée à traiter l'ostéoporose selon l'une quelconque des revendications précédentes, le vêtement orthopédique (2, 2') étant coupé sous la forme d'un sous-vêtement en une ou en plusieurs parties, notamment d'un dénommé body (24, 24'), d'une combinaison de cycliste, d'un maillot, notamment d'un t-shirt ou d'un gilet.

15. Orthèse modulaire (1'), destinée à traiter l'ostéoporose selon l'une quelconque des revendications précédentes, pour recevoir de manière amovible ou inamovible et pour guider le système de sangles (8'), le vêtement orthopédique (2') étant conçu au moins par endroits en plusieurs couches, de préférence en deux couches, de sorte que le système de sangles (8') soit positionnable au moins en partie entre les deux couches du vêtement (2').
